# EUROPEAN PATENT APPLICATION

(11) **EP 0 702 073 A1**
(43) Date of publication of application: **20.03.1996**
(21) Application number: 95306580.2
(22) Date of filing: 19.09.1995
(51) Int. Cl.: C09K 7/02, C07H 15/04

(54) **Shale stabilising drilling fluid employing saccharide derivatives**

(30) Priority: 19.09.1994 US 309002
(71) Applicant: BAKER HUGHES INCORPORATED, Houston, Texas 77027 (US)
(72) Inventor: Clapper, Dennis K., Houston, Texas 77072 (US); Watson, Stephen K., Houston, Texas 77077 (US)
(74) Representative: Woodman, Derek

(57) **Abstract**

An additive for water based drilling fluids comprises saccharide derivatives formed from the reaction product between saccharides or saccharide derivatives (e.g., methyl glucoside) and at least one of the following classes of compounds or compounds (1) alcohols, (2) alkyl halides, (3) alkylene oxides, (4) epichlorohydrin or (5) amines. Particularly desirable are derivatives of alkyl glycoside with the listed chemicals. Especially desirable are reaction products of methyl glucoside with the listed chemicals. This additive is well suited for stabilizing a subterranean formation penetrated by a borehole and having water sensitive shales, clays or fines. A particularly preferred drilling fluid additive is the reaction product of methyl glucoside and such alkylene oxides as ethylene oxide, propylene oxide and/or butylene oxide. Such an additive will impart to a drilling fluid temperature activation whereby the additive will be soluble in water at ambient conditions (e.g., surface conditions during well drilling operations), but become insoluble at elevated downhole temperatures. In the process of becoming insoluble at elevated temperatures, these compounds will concentrate at important surfaces such as the drill bit cutting surface, the borehole surface and the surfaces of the drilled cuttings.

## Description

### Field of the Invention:

This invention relates to a drilling fluid composition and method of drilling a subterranean well to inhibit the absorption of aqueous medium by shales in aqueous alkaline environments. More particularly, this invention relates to a method and composition for reducing shale swelling where the shale is stabilized using a saccharide derivative. A preferred embodiment employs the reaction product between alkyl glucoside and alkylene oxides to form a temperature activated drilling fluid additive.

### Background of the Invention:

A rotary system is a common form of drilling a subterranean well. This system depends upon the rotation of a column of drill pipe to the bottom to which is attached a drilling bit. The drill bit cuts into the earth, causing cuttings. A drilling fluid must be used to carry these cuttings to the surface for removal to allow the bit to continue functioning and the bottom of the hole to be kept clean and free of cuttings.

Aqueous based drilling fluids are frequently utilized in the drilling, completion and workover operations of subterranean wells. Such drilling fluids utilize clear water, brine, or sea water as the primary liquid phase, to which may be added known dispersants or deflocculants, filtration control agents, viscosifiers and suspending and weighting agents, such as bentonite and barite, respectively.

In most areas, the major portions of the formations penetrated while drilling a well are shale. By shale it is meant materials such as bentonite and the like, claystones and gumbo-type colloidal-clay substances and related substances which possess the property of hydrodynamic volume increase when exposed to aqueous environments. These water sensitive shales hydrate in water rather easily and may swell to many times their original size. Swelling herein refers to the hydrodynamic volume increase of the shale.

Swelling of shales is believed to be attributable to surface hydration, interlayer swelling and osmotic swelling. Surface hydration is adsorption of water onto the shale surfaces and is particularly active in shales because of high surface area. Shales, particularly smectites, may have a lattice-type structure which allows the water to be absorbed between layers as well as upon the particle surface. This absorbed water is considered held by hydrogen bonding to basal surfaces which, because of atomic structure normally have a net negative charge. On the other hand, osmotic swelling occurs because the concentration of cations adsorbed on the basal surface of the clays and held at surface edges is greater than in the liquid itself. This force draws the liquid into the shale particle. Of course, the degree of the osmotic effect depends on the salt concentrations both in the shale particle and in the liquid.

Drilling of water sensitive shales with conventional water-base drilling fluids has long been plagued with problems. The shale formation penetrated may swell and slough as a result of interaction with the drilling fluid, and cave in, leading to hole enlargement and serious mechanical difficulties. Further, interaction between water and water sensitive shale usually has an adverse effect on drilling fluids, because the shale disperses into colloidal sized particles of clay which tend to increase the viscosity and density of the drilling fluids to such an extent that they are unusable without considerable dilution and chemical treatment. These difficulties have led to efforts to inhibit shale swelling during drilling. Inhibition of swelling herein means the ability of a process to retard the hydration of shales so they remain substantially cohered and basically substantially in their original size, shape and volume.

One early method of inhibiting the swelling of shale when drilling subterranean wells with a water base drilling fluid was the use of calcium-treated muds. The polyvalent calcium cation holds the clay layers together, it is thought, by bonding to the clay surfaces in competition with water molecules. Calcium is provided by lime hydrate, gypsum and calcium chloride, and occurs in sea water. Calcium treated muds and sea water require thinners such as lignosulfonate, because the divalent calcium cation is flocculating. However, in deep hot holes, calcium treated muds tend to setup or become so highly gelled they cannot be circulated out of the borehole.

The so-called polymer muds have come into later favor, generally relying on a combination of polymers and soluble salts to inhibit swelling and dispersion. Commonly used polymers are cellulose derivatives, partially hydrolyzed polyacrylamide ("PHPA"), xanthan gum and biopolymers. The commonly used polymers are anionic polyelectrolytes and are thought to be adsorbed at positive charge sites on the edges of layer surfaces at multiple places along the chain length of the polymer, thereby binding the clay particles together and encapsulating cuttings. Soluble salts are needed in these muds to allow cations to react with the net negatively charged layer surfaces and thereby reduce the repulsing forces between clay surfaces. Potassium salts (potassium chloride is the usual salt preferred) are considered more effective at repressing swelling and dispersion than sodium salts. For example, see U.S. Patents 3,738,437 and 4,664,818. However, these polymer mud systems are now less available. Environmental regulations restricting release of chlorides into on-shore waters are increasingly limiting use of alkali metal chlorides to offshore drilling muds, where chlorides release into naturally salty sea water is not restricted.

While the foregoing polymer-based mud systems are now frequently used in rotary drilling operations, such mud systems, including those based on PHPA suffer from certain disadvantages. For example, such polymer-based drilling fluids can exhibit (1) poor tolerance for solids contamination; (2) degradation and reduction in performance at high temperature conditions (e.g., bottom hole temperatures); and (3) polymer hydrolysis and associated poor performance at high pH.

Methyl glucoside has been suggested for use as a drilling fluid additive and is believed to be particularly useful in shale stabilization. Methyl glucoside is potentially an environmentally acceptable alternative to oil base drilling muds and therefore is a potentially important product for use in drilling for oil and natural gas. However, methyl glucoside does suffer from several deficiencies. For example, methyl glucoside is expensive relative to other conventional drilling fluids and additives. This high expense is exacerbated by the fact that the methyl glucoside must be used in relatively high concentrations (e.g., 30% by volume) leading to an even higher, and perhaps prohibitive cost for such drilling fluids.

### Summary of the Invention:

The above-discussed and other problems and deficiencies of the prior art are overcome or alleviated by the method and composition for providing enhanced shale stabilization of the present invention. In accordance with the present invention, an additive for water based drilling fluids comprises a saccharide derivative which constitutes the reaction product between a saccharide or saccharide derivative and at least one of alcohols, alkyl halides, alkylene oxides, epichlorohydrin and/or amines. The resultant reaction product is particularly effective as a shale inhibitor and exhibits an important advantage over prior art use of methyl glucoside in that shale inhibition is achieved with relatively lower concentrations of the saccharide derivative (as compared to the methyl glucoside, per se). This ability to utilize lower concentrations will, in turn, significantly lower the overall cost (again, as compared to the use of methyl glucoside, per se) for the drilling fluids made with the saccharide derivative.

In accordance with another embodiment of the present invention, there is provided a well fluid comprising an aqueous component and including the well fluid additive described above.

In accordance with still another embodiment of the present invention there is provided a method of stabilizing a subterranean formation penetrated by a borehole and having water sensitive shales, clays or fines. The method generally includes introducing a formation treatment fluid into the borehole and into contact with the formation, wherein the formation treatment fluid comprises the well fluid additive described above.

A particularly preferred embodiment of the present invention comprises a temperature activated drilling fluid additive composed of the reaction product between alkyl glycoside and alkylene oxides such as ethylene oxide, propylene oxide and/or butylene oxide. Such alkyl glycoside derivatives are characterized as being "temperature activated" as a result of these compounds being soluble at ambient conditions (e.g., surface conditions during well drilling operations), but becoming insoluble at elevated downhole temperatures. In the process of becoming insoluble (via a cloud point phenomenon), these compounds will concentrate at important surfaces such as (1) the cuttings surface (so as to inhibit hydration and dispersion of shales); (2) the bit surface (so as to lubricate and prevent bit balling); and (3) the borehole (so as to improve borehole stability and lubricity as well as acting to decrease fluid loss). Because this alkyl glycoside derivative will concentrate at the critical areas of concern during the drilling operation, lower percentages of the derivative will be required (as compared to a scenario where no concentration at elevated temperature takes place); and therefore the alkyl glycoside derivative of this invention is low cost. Moreover, because such alkyl glycoside derivatives do not cause environmental concerns, these compounds provide a cost effective and environmentally advantageous alternative to oil-based drilling muds.

The above-discussed and other features and advantages of the present invention will be appreciated and understood by those skilled in the art from the following detailed description.

### Description of the Preferred Embodiment:

This invention presents a shale swelling inhibitor (e.g., stabilizing composition) comprising a well fluid additive. This additive comprises a saccharide (e.g., alkyl glycoside) derivative which constitutes the reaction product between a saccharide or saccharide derivative and at least one of alcohols, alkyl halides, alkylene oxides, epichlorohydrin and/or amines. The resultant reaction product is particularly effective as a shale inhibitor and exhibits an important advantage over prior art use of methyl glucoside, per se, in that shale inhibition is achieved with relatively lower concentrations of the saccharide derivative (as compared to the methyl glucoside, per se). This ability to utilize lower concentrations will, in turn, significantly lower the overall cost (again, as compared to the use of methyl glucoside, per se) for the drilling fluids made with the saccharide derivative.

In the practice of the drilling method of the present invention, the drillstring is rotated to cut a borehole into the earth while circulating a drilling fluid down through the drillstring and then up the annulus between the drilling string and the wall of the borehole. The drilling fluid utilized in the drilling method of the present invention will comprise an aqueous component and the above described additive of the present invention.

The aqueous medium employed in the well fluids of the present invention may be any kind of water from any source including, but not limited to, fresh water, sea water, water from the subterranean reservoir, or a natural or synthetic brine.

The saccharide derivatives of the present invention are derived from sugars including monosaccharides and oligosaccharides consisting of up to ten sugar units. Monosaccharide derivatives may be derived from sugars having 3 to 10 carbon atoms, e.g., erythrose, xylose, galactose (Structures 1 to 3). Oligosaccharides may include lactose, maltose or sucrose (Structures 4 to 6). Particularly suitable for further derivatization are alkyl glycosides (Structure 8). Alkyl glycosides are prepared by the reaction of an aldose such as glucose (Structure 7) with alcohol and a catalyst which is often mineral acid (Equation 1). Especially desirable for further reaction is methyl glucoside (MEG).

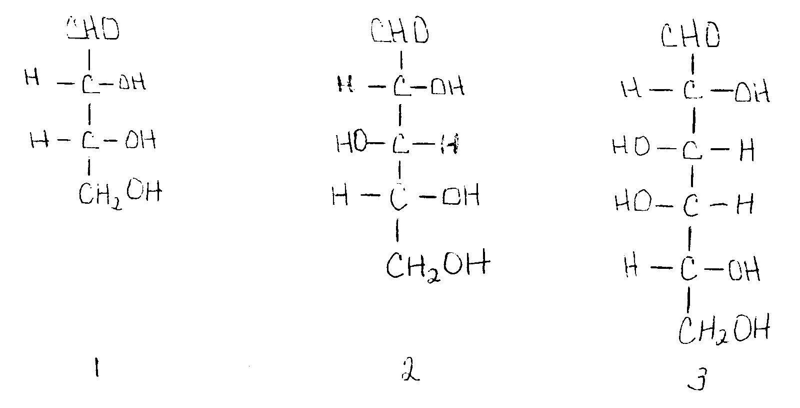

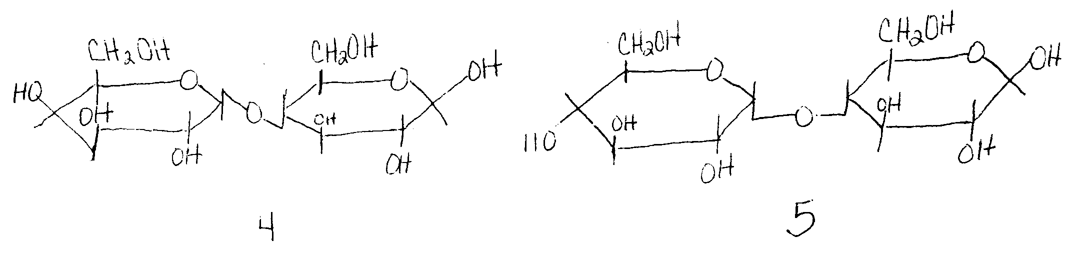

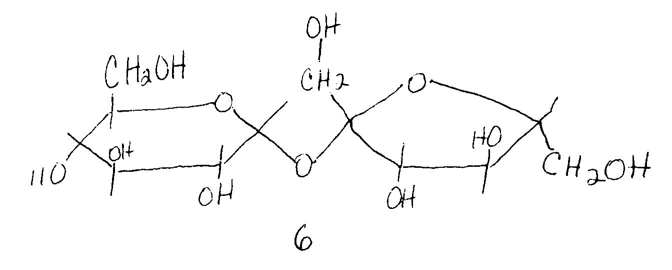

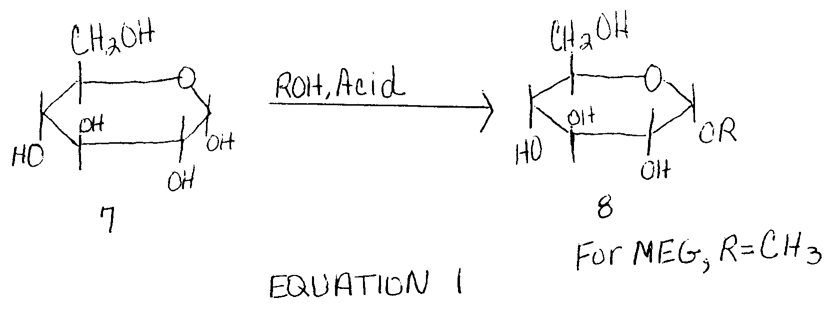

Each of these structures may be derivatized by reaction with at least one of (1) alkyl halides, (2) epichlorohydrin, (3) amines, (4) alcohols and (5) alkylene oxides.

The saccharide derivatives of the present invention may be categorized, for purposes of convenience, as the reaction products of a saccharide such as glucose, or a saccharide derivative, such as a MEG, with at least one of (1) alkyl halides, (2) epichlorohydrin, (3) amines, and (4) alkylene oxides. In addition, aldoses such as glucose may be reacted with (5) alcohols to produce alkyl glycosides such as MEG which may be further derivatized by reactions as discussed in examples (1) to (4). For all examples it is understood that reaction of the saccharide or saccharide derivative occurs primarily at unsubstituted hydroxyl groups (OH) and that multiple substitution is possible (i.e., reaction may occur at more than one hydroxyl site.)

### (1) Alkyl Halides

Glucose (GLU) or MEG may be reacted with alkyl halides of 1 to 18 carbon atoms including but not limited to methyl iodide, ethyl bromide or dodecyl chloride as follows: 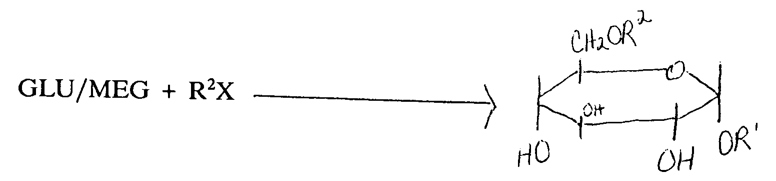
where R¹ is H or CH₃; and R is H or alkyl groups and X is chlorine, bromine or iodine.

### (2) Epichlorohydrin

Glucose or MEG may be reacted with epichlorohydrin as follows:

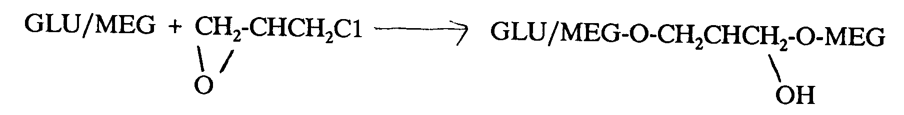

### (3) Amines

Glucose or MEG may be reacted with amines such as diethylaminoethyl chloride or 2,3-epoxypropyl-trimethylammonium chloride, as follows: 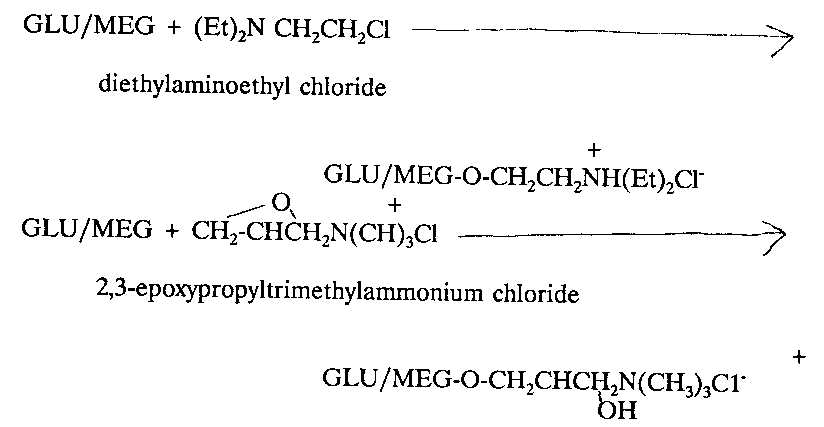
It will be appreciated that the foregoing MEG/amine or GLU/amine derivatives will provide a low molecular weight cationic shale stabilizer.

### (4) Alkylene Oxide

Glucose or MEG may be reacted with alkylene oxides such as ethylene oxide, propylene oxide or butylene oxide or combinations of ethylene oxide with propylene or butylene oxide as follows: 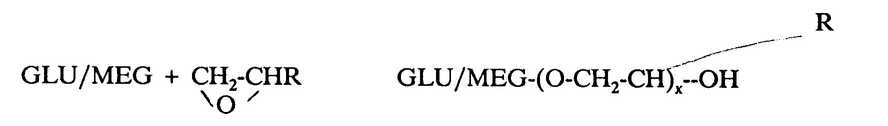
where R is H, CH₃, or CH₂ CH₃ and x is one or greater.

The foregoing methyl glucoside derivatives (based on MEG and alkylene oxides) are characterized as being "temperature activated" as a result of these compounds being soluble at ambient conditions (e.g., surface conditions during well drilling operations), but insoluble at elevated downhole temperatures. In the process of becoming insoluble (via a cloud point phenomenon), these compounds will concentrate at important surfaces such as (1) the cuttings surface (so as to inhibit hydration and dispersion of shales); (2) the bit surface (so as to lubricate and prevent bit balling); and (3) the borehole (so as to improve borehole stability and lubricity as well as acting to decrease fluid loss).

The MEG/alkylene oxide derivatives can be described in terms of a characteristic "cloud point" in the range of from about 80°F to about 212°F. The "cloud point" for a material is determined by preparing a fluid comprising a predetermined percent by volume of the water soluble MEG derivative compound in a water medium, which may be fresh water, sea water, natural brine, or synthetic brine and heating the solution until the solution becomes cloudy. The cloud point is that temperature at which the material begins to come out of solution, becoming immiscible in the water and forming as a separate liquid phase. This characteristic of the MEG/alkylene oxide derivative compounds in the additive composition of this invention permits the compound to be soluble in water under most surface conditions in conformity with environmental regulations which do not permit an oil "sheen" to be left upon receiving waters by a discharged drilling fluid, yet behave like an oil at hotter temperatures in the borehole at and above the cloud point of the selected MEG derivative compound. As a result, a relatively low 1 to 10 percent by volume concentration of the MEG/alkylene oxide derivative may be employed.

Thus, because these methyl glucoside derivatives will concentrate at the critical areas of concern during the drilling operation, lower percentages of the derivative will be used (as compared to a scenario where no concentration at elevated temperature takes place); and therefore the methyl glucoside derivative of this invention is low cost. Moreover, because such methyl glucoside derivatives do not cause environmental concerns, these compounds provide a cost effective and environmentally advantageous alternative to oil based drilling muds.

### (5) Acids and Alcohols

Aldoses such as glucose may be reacted with alcohols other than methanol, including ethanol, propanol, isopropanol, butanol, octadecanol to form an alkylglucoside as follows: 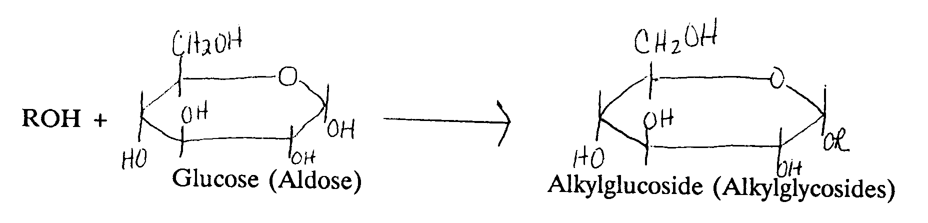
Where R contains 2 to 18 carbons atoms (but not CH₃).

Alkyl glycosides may be further derivatized by reactions cited in examples (1) to (4).

It is also to be understood that other additives used by those skilled in the art may also be added to the drilling fluids of the present invention, as long as they do not have a substantial detrimental effect on the well fluid, including but not limited to, for example, surfactants, weighting materials, breakers, loss circulation additives and salts.

The relative amounts of the components of the well fluid additive of the present invention are generally selected to provide compatibility with each other and with the well fluid, and to provide suitable shale, clay or fines stabilization.

Generally based on the total volume of the well fluid additive, the well fluid additive of the present invention will comprise in the range of about 0.5 to about 10 volume percent saccharide derivative. Preferably, the well fluid comprises in the range of about 0.5 to about 10 volume percent methyl glucoside derivative and most preferably in the range of about 1 to about 3 volume percent methyl glucoside derivative.

In the practice of the present invention, the well fluid additive is generally added to the well fluid in an amount in the range of about 2 to about 15 pounds/bbl well fluid.

While preferred embodiments have been shown and described, various modifications and substitutions may be made thereto without departing from the spirit and scope of the invention. Accordingly, it is to be understood that the present invention has been described by way of illustrations and not limitation.

## Claims

1. A well fluid comprising an aqueous component and a sacchaaride derivative component derived from the reaction product of a saccharide or saccharide derivative and at least one of the group consisting of alkyl halides, epichlorohydrin, amines, alkylene oxides and alcohols.

2. A well fluid as claimed in claim 1 wherein said saccharide derivative component is present in an amount effective to enhance shale stabilization.

3. A well fluid as claimed in claim 1 or 2 wherein said saccharide derivative component is present in an amount of from about 0.5 to about 10 volume percent of the total well fluid.

4. A well fluid as claimed in claim 1 wherein said saccharide derivative component is derived from a sugar or sugar derivative having up to 10 sugar units.

5. A well fluid as claimed in claim 4 wherein said sugar is selected from the group consisting of monosaccharides and oligosaccharides.

6. A well fluid as claimed in claim 5 wherein said monosaccharide sugar has 3 to 10 carbon units.

7. A well fluid as claimed in claim 6 wherein said monosaccharide sugar is selected from the group consisting of erythrose, xylose and galactose.

8. A well fluid as claimed in claim 5 wherein said oligosaccharide sugar is selected from the group consisting of lactose, maltose and sucrose.

9. A well fluid as claimed in any preceding claim wherein said saccharide derivative comprises at least one alkyl glycoside.

10. A well fluid claim as claimed in claim 9 wherein said alkyl glycoside comprises methyl glucoside.

11. A well fluid as claimed in claim 1 wherein said saccharide comprises glucose or a derivative thereof.

12. A well fluid as claimed in claim 1 wherein said saccharide derivative component is the reaction product of glucose or methyl glucoside and a compound of formula RX (wherein R is H or an alkyl group of 1-18 carbon atoms and X is chlorine, bromine or iodine).

13. A well fluid as claimed in claim 12 wherein RX is selected from the group consisting of methyl iodide, ethyl bromide or dodecyl chloride.

14. A well fluid as claimed in claim 1 wherein said saccharide derivative component is the reaction product of glucose or methyl glucoside and (Et) ₂NCH₂CH₂Cl.

15. A well fluid as claimed in claim 1 wherein said saccharide derivative component is the reaction product of glucose or methyl glucoside and

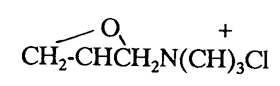

16. A well fluid as claimed in claim 1 wherein said saccharide derivative component is the reaction product of glucose or methyl glucoside and 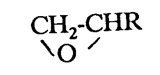 where R is H, CH₃ or CH₂CH₃.

17. A well fluid comprising an aqueous component and an alkyl glycoside other than methyl glucoside.

18. A well fluid comprising an aqueous component and an alkyl glucoside derivative component derived from the reaction product of an alkyl glucoside other than methyl glucoside and at least one of the group consisting of alkyl halides, epichlorohydrin, amines and alkylene oxides.

19. A method of stabilizing a subterranean formation penetrated by a borehole wherein the formation comprises water-sensitive materials, the method comprising introducing a formation treatment fluid into the borehole and into contact with the formation, wherein the formation treatment fluid comprises a well fluid as claimed in any preceding claim.

20. Use of an additive in water based drilling fluids to provide stabilisation of shale, said additive comprising a saccharide derivative component as defined in claim 1.
